# EUROPEAN PATENT APPLICATION

(11) **EP 4 635 959 A1**
(43) Date of publication of application: **22.10.2025**
(21) Application number: 23903851.6
(22) Date of filing: 01.12.2023
(51) Int. Cl.: C07F 3/02, A61K 31/555, A61P 25/00, A61P 25/28

(54) **NOVEL CRYSTALLINE FORM OF MAGNESIUM-SERINATE AND METHOD FOR PRODUCING SAME**

(30) Priority: 16.12.2022 KR 20220177178
(71) Applicant: Astrogen, Inc., Daegu 41072 (KR)
(72) Inventor: HWANG, Su-Kyeong, Daegu 41072 (KR); RYU, Hyung Chul, Daegu 41072 (KR); KIM, Yong-Dae, Daegu 41072 (KR); KIM, Joon Hyuk, Daegu 41072 (KR); SUN, Mijin, Daegu 41072 (KR); PARK, Seong Hyeok, Daegu 41072 (KR); KWAK, Soyoung, Daegu 41072 (KR); YANG, Hyun Jun, Daegu 41072 (KR)
(74) Representative: Potter Clarkson
(86) International application number: PCT/KR2023/019699
(87) International publication number: WO 2024/128648

(57) **Abstract**

The present invention relates to a new crystalline form of magnesium-serinate represented by chemical formula 1 and a method for producing same. The crystalline form of the present invention has excellent heat and moisture stability and can be preserved stably without undergoing decomposition or crystalline form transition even during the long-term storage and thus can be advantageously used as a pharmaceutical raw material for medications.

## Description

### Technical Field

The present invention relates to a novel crystalline form of magnesium-serinate and a preparation method thereof.

### Background Art

Serine (L-serine) has traditionally been considered a nonessential amino acid and may be used in the synthesis of glutathione as a precursor to glycine and cysteine. It is known that serine mediates neuroprotective effects in the mammalian central nervous system and reduces age-related oxidative stress and inflammatory responses in the hypothalamus when ingested at an appropriate concentration for a long period of time. Additionally, it has recently been reported that functional foods containing a high concentration of serine have an antioxidant effect.

Magnesium (Mg) is an essential mineral used as a cofactor in the human body and is involved in more than 300 biochemical reactions necessary to maintain homeostasis. Additionally, it not only induces the production of nucleic acids and participates in the ATP reaction, but also has a wide range of physiologically diverse functions, such as insulin secretion, muscle contraction, and regulation of intracellular calcium concentration.

It has been reported that the intake of magnesium in the Western population is insufficient, and insufficient intake of magnesium is known to be associated with the induction of hypertension, cardiovascular disease, and type 2 diabetes. Additionally, magnesium intake is known to be effective for mild and moderate depression in adults. Clinical results have been reported that taking 4 tablets of 500 mg magnesium chloride daily results in an intake of 248 mg of elemental magnesium, which significantly reduces symptoms of depression and anxiety regardless of age, severity of depression, and whether or not antidepressants are taken.

Meanwhile, Korean Patent No. 10-1952443 (Patent Document 1) reports a compound represented by Formula 2 below in which a magnesium atom is chelated to L-serine.

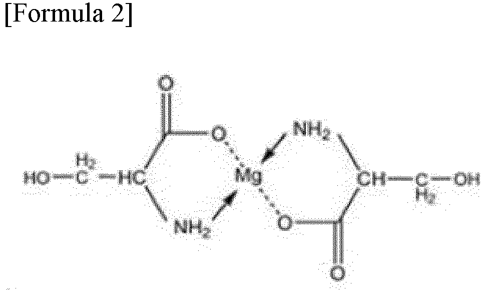

The compound of Formula 2 above has a structure in which two serine molecules are bonded to a single magnesium atom via an ionic bond and a coordinate bond, and each carboxylic acid group (COO⁻) of two serine molecules forms an ionic bond with the magnesium atom, and the two amine groups (-NH₂) forms coordinate bonds. The compound of Formula 2 above can be obtained by reacting L-serine with magnesium oxide (MgO) or magnesium hydride (MgH₂), and water (H₂O) or hydrogen (H₂) is generated as a by-product, respectively.

Due to a very high-water absorption rate, the compound of Formula 2 above exhibits stability degradation, including discoloration and denaturation, when stored at room temperature. These issues necessitate stringent storage and formulation requirements, such as refrigeration, which makes its application in mass pharmaceutical production unfavorable.

### [Prior Art Documents]

### [Patent Documents]

(Patent Document 0001) Korean Patent No. 10-1952443

### [Non-Patent Documents]

(Non-Patent Document 0001) Zhou X, Zhang H, He L, Wu X and Yin Y (2018) Long-Term L-Serine Administration Reduces Food Intake and Improves Oxidative Stress and Sirt1/NFkB Signaling in the Hypothalamus of Aging Mice. Front. Endocrinol. 9:476.
(Non-Patent Document 0002) Zhou X, He L, Zuo S, Zhang Y, Wan D, Long C, et al. Serine prevented high-fat diet-induced oxidative stress by activating AMPK and epigenetically modulating the expression of glutathione synthesis-related genes. Biochim Biophys Acta (2018) 1864:488-98.
(Non-Patent Document 0003) Metcalf JS, Dunlop RA, Powell JT, Banack SA, Cox PA. L-Serine: a naturally-occurring amino acid with therapeutic potential. Neurotox Res. (2018) 33:213-21.
(Non-Patent Document 0004) Dunlop RA, Powell JT, Metcalf JS, Guillemin GJ, Cox PA. L-serine-mediated neuroprotection includes the upregulation of the ER stress chaperone protein disulfide isomerase (PDI). Neurotox Res. (2018) 33:113-22.
(Non-Patent Document 0005) Tarleton EK, Littenberg B, MacLean CD, Kennedy AG, Daley C (2017) Role of magnesium supplementation in the treatment of depression: A randomized clinical trial. PLoS ONE 12(6): e0180067.
(Non-Patent Document 0006) Swaminathan R. Magnesium metabolism and its disorders. Clin. Biochem. Rev. 2003; 242:47-66.
(Non-Patent Document 0007) Topf J.M., Murray P.T. Hypomagnesemia and hypermagnesemia. Rev. Endocr. Metab. Disord. 2003; 42:195-206.
(Non-Patent Document 0008) King D.E., Mainous A.G., 3rd, Geesey M.E., Woolson R.F. Dietary magnesium and C-reactive protein levels. J. Am. Coll. Nutr. 2005; 243:166-171.
(Non-Patent Document 0009) Song Y., Sesso H.D., Manson J.E., Cook N.R., Buring J.E., Liu S. Dietary magnesium intake and risk of incident hypertension among middle-aged and older US women in a 10-year follow-up study. Am. J. Cardiol. 2006; 9812:1616-1621.
(Non-Patent Document 0010) Song Y.Q., Manson J.E., Cook N.R., Albert C.M., Buring J.E., Liu S. Dietary magnesium intake and risk of cardiovascular disease among women. Am. J. Cardiol. 2005; 968:1135-1141.
(Non-Patent Document 0011) Song Y.Q., Manson J.E., Buring J.E., Liu S. Dietary magnesium intake in relation to plasma insulin levels and risk of type 2 diabetes in women. Diabetes Care. 2004; 271:59-65

### Disclosure of Invention

### Technical Problem

An object of the present invention is to provide a novel crystalline form of a magnesium-serinate compound and a method for preparing the same.

An object of the present invention is to provide a pharmaceutical composition for preventing or treating developmental disorders and/or neurodegenerative diseases comprising a crystalline form of a magnesium-serinate compound.

An object of the present invention is to provide a health functional food for preventing or improving developmental disorders and/or neurodegenerative diseases, comprising a crystalline form of a magnesium-serinate compound.

An object of the present invention is to provide a use of a crystalline form of a magnesium-serinate compound for the prevention and/or treatment of developmental disorders and/or neurodegenerative diseases.

An object of the present invention is to provide a method for preventing or treating developmental disorders and/or neurodegenerative diseases, comprising administering a crystalline form of a magnesium-serinate compound to an individual.

An object of the present invention is to provide a use of a crystalline form of a magnesium-serinate compound for the preparation of a medicament for preventing or treating developmental disorders and/or degenerative neurological diseases.

### Solution to Problem

The present inventors have found that the novel crystalline form of magnesium-serinate compound not only exhibits excellent physical properties, stability, and low hygroscopicity, but is also thermodynamically stable, and thus suitable for use as a pharmaceutical raw material because it does not undergo discoloration, denaturation, or polymorphic transition caused by aging during storage, thereby completing the present invention.

Hereinafter, the present invention will be described in more detail. In the present specification, when a part is said to "comprise" a certain component, it means that it may further include other components instead of excluding other components unless otherwise stated.

### Crystalline form

The present invention provides a crystalline form of a magnesium-serinate compound represented by Formula 1 below:

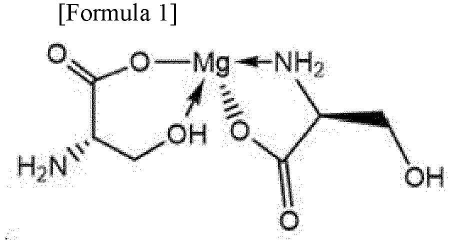

The compound of Formula 1 has a chemical name of magnesium-di-(2S)-2-amino-3-hydroxypropanoate, and in the present invention, it is named "magnesium-L-serinate" or "magnesium-serinate".

The magnesium-serinate compound represented by Formula 1 of the present invention has a structure in which two L-serine molecules form an ionic bond and a coordinate bond with one magnesium (Mg) atom. Specifically, in the magnesium-serinate compound represented by Formula 1, the carboxyl group of two molecules of L-serine and the magnesium atom are ionically bonded, whereas the hydroxy group of one molecule of L-serine and the amine group of the other molecule of L-serine form a coordinate bond with the magnesium atom.

That is, the crystalline form of the compound represented by Formula 1 is a crystalline solid compound composed of a structure in which two L-serine molecules form an ionic bond and a coordinate bond with one magnesium atom, as shown in the following structural formula.

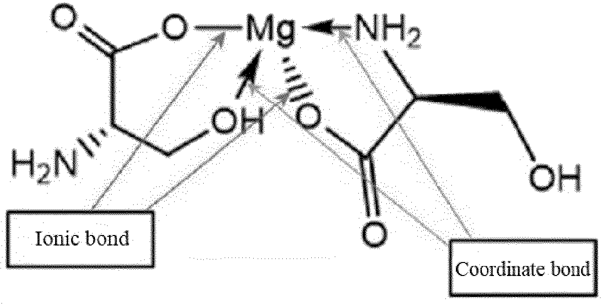

The crystalline form of the magnesium-serinate compound represented by Formula 1 of the present invention is a novel crystalline compound, which is a sufficiently secured crystalline form that is stable against heat and moisture, and has stability in that the active ingredient does not decompose or polymorphic transition occurs during production, formulation, storage, and distribution, thus being advantageous for drug production.

In an embodiment of the present invention, the crystalline form of the magnesium-serinate compound represented by Formula 1 may include, in the X-ray powder diffraction pattern, diffraction peaks at diffraction angles (2θ±0.2°) of 13.53°, 16.00°, and 22.05°; and three or more diffraction peaks at diffraction angles (2θ±0.2°) selected from the group consisting of 18.36°, 19.75°, 20.00°, 20.95°, 24.39°, and 28.37°.

Additionally, in an embodiment of the present invention, the crystalline form of the magnesium-serinate compound represented by Formula 1 may further include, in the X-ray powder diffraction pattern, one or more diffraction peaks at diffraction angles (2θ±0.2°) selected from the group consisting of 18.89°, 25.73°, 26.39°, 29.55°, 30.07°, 32.39°, 33.51°, 33.99°, 34.42°, and 40.36°, in addition to the diffraction peaks described above.

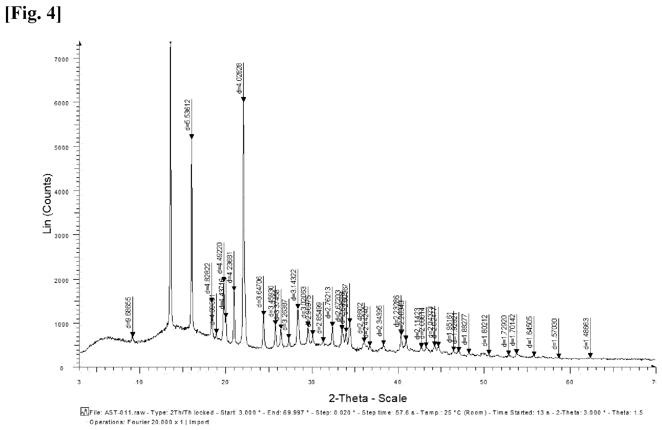

Additionally, the crystalline form of the magnesium-serinate compound represented by Formula 1 of the present invention may further include in the X-ray powder diffraction pattern the diffraction peak shown in Fig. 4.

In an embodiment of the present invention, the crystalline form of the magnesium-serinate compound represented by Formula 1 may have a maximum endothermic peak at 283±3°C when analyzed by the differential scanning calorimetry (DSC).

That is, the crystalline form of the present invention has excellent thermal stability with a thermal decomposition temperature of 283±3°C, and can be stably maintained without decomposition of the active ingredient even during long-term storage.

The present invention provides a magnesium-serinate compound, which is a crystalline form of magnesium-serinate compound in which two serine molecules are bonded to one atom of magnesium, and which includes in the X-ray powder diffraction pattern diffraction peaks at diffraction angles (2θ±0.2°) of 13.53°, 16.00°, and 22.05°.

Additionally, in an embodiment of the present invention, the crystalline form of the magnesium-serinate compound, in which two serine molecules are bonded to one atom of magnesium, may further include three or more diffraction peaks in the X-ray powder diffraction pattern at diffraction angles (2θ±0.2°) selected from the group consisting of 18.36°, 19.75°, 20.00°, 20.95°, 24.39°, and 28.37°.

Additionally, in an embodiment of the present invention, the crystalline form of the magnesium-serinate compound, in which two serine molecules are bonded to one atom of magnesium, may further include one or more diffraction peaks in the X-ray powder diffraction pattern at diffraction angles (2θ±0.2°) selected from the group consisting of 18.89°, 25.73°, 26.39°, 29.55°, 30.07°, 32.39°, 33.51°, 33.99°, 34.42°, and 40.36°.

In this case, the crystalline form of the magnesium-serinate compound according to the present invention may have a maximum endothermic peak at 283±3°C when analyzed by the differential scanning calorimetry (DSC).

In the present invention, the magnesium-serinate compound, which is a crystalline form of magnesium-serinate compound in which two serine molecules are bonded to one atom of magnesium, and which includes in the X-ray powder diffraction pattern diffraction peaks at diffraction angles (2θ±0.2°) of 13.53°, 16.00°, and 22.05°, may refer to the same crystalline form as that of the magnesium-serinate compound represented by Formula 1.

### Method for preparing crystalline form of compound represented by Formula 1

As a method for preparing a crystalline form of the compound represented by Formula 1, the present invention provides a method for preparing a crystalline form of the magnesium-serinate compound, comprising:
a) mixing L-serine, magnesium (Mg) or magnesium oxide, and a solvent and stirring the mixture; and
b) filtering the crystals produced after the stirring.

In step a), magnesium (Mg) may be magnesium in powder or granular form. The magnesium oxide may be a divalent oxide which is a stable form of magnesium, and specifically, may be one or more selected from the group consisting of magnesium methoxide (Mg(OCH₃)₂), magnesium ethoxide (Mg(OC₂H₅)₂), magnesium chloride (MgCl₂), magnesium bromide (MgBr₂), magnesium carbonate (MgCO₃), and magnesium citrate (C₆H₆O₇Mg). More specifically, the magnesium oxide may be magnesium methoxide or magnesium ethoxide. Commercially available products may be used as the magnesium or magnesium oxide.

The solvent may be a polar solvent, and specifically, as an alcohol solvent having 1 to 9 carbon atoms, it may be at least one selected from the group consisting of methanol, ethanol, isopropanol, and *tert*-butanol. More specifically, it may be methanol or ethanol.

The stirring in step a) may be performed at room temperature (1°C to 30°C) or may be reflux stirring at a temperature range of ± 20°C of the boiling point of the solvent used. Additionally, stirring in step a) may be performed vigorously until L-serine and magnesium or magnesium oxide are completely dissolved in the solvent, and may proceed for 1 to 120 hours.

In step b), the filtration may be performed at room temperature using a Büchner funnel but is not limited thereto.

The preparation method of the present invention may further include the steps of washing and drying the crystals produced in step b) after the step of filtering the same.

The washing may be performed using a reaction solvent cooled to room temperature. In particular, as the reaction solvent, at least one selected from the group consisting of methanol, ethanol, isopropanol, and *tert*-butanol may be used, and specifically, methanol or ethanol may be used.

The drying may be performed in a temperature range from room temperature (1°C to 30°C) to 120°C.

### A pharmaceutical composition or health functional food comprising a crystalline form of compound represented by Formula 1

In a still another embodiment, the present invention provides a pharmaceutical composition comprising a crystalline form of the compound represented by Formula 1 above.

In a still another embodiment, the present invention provides a pharmaceutical composition for preventing or treating developmental disorders or neurodegenerative diseases, comprising the crystalline form of the compound represented by Formula 1 as an active ingredient.

In the present invention, the developmental disorder may be one or more diseases selected from the group consisting of autism spectrum disorder (ASD), attention deficit hyperactivity disorder (ADHD), intellectual disability, language disorder, motor disorder including motor development delay, tic disorder, and Tourette syndrome.

In the present invention, the neurodegenerative disease may be one or more diseases selected from the group consisting of Alzheimer's disease, vascular dementia, mild cognitive impairment, Parkinson's disease, Huntington's disease, amyotrophic lateral sclerosis, multiple sclerosis, and cerebellar degenerative diseases.

In the present invention, the pharmaceutical composition may be formulated into one or more formulations selected from the group consisting of powders, granules, tablets, capsules, suspensions, emulsions, syrups, aerosols, suppositories, eye drops, and injections.

The content of the crystalline form as an active ingredient in the pharmaceutical composition of the present invention may be calculated in consideration of the patient's age, weight, *etc.* In general, it may be included in 0.1 wt% to 80.0 wt% based on the total weight of the pharmaceutical composition.

Additionally, the pharmaceutical composition of the present invention may include pharmaceutically acceptable additives, such as excipients, diluents, binders, disintegrants, glidants, pH adjusters, antioxidants, and solubilizing agents, within the range that does not impair the effects of active ingredients. Examples of pharmaceutically acceptable additives that may be used in formulating the pharmaceutical composition of the present invention include microcrystalline cellulose, xylitol, erythritol, methylcellulose, polyvinylpyrrolidone, starch, acacia, alginate, gelatin, lactose, dextrose, sucrose, propylhydroxybenzoate, cellulose, methylhydroxybenzoate, magnesium stearate, talc, sorbitol, mannitol, maltitol, calcium phosphate, calcium silicate, crospovidone, croscarmellose sodium, hypromellose, mineral oil, *etc.,* but are not limited thereto.

Additionally, the pharmaceutical composition of the present invention may be formulated into one or more formulations selected from the group consisting of powders, granules, tablets, capsules, suspensions, emulsions, syrups, aerosols, suppositories, eye drops, and injections according to conventional formulation methods, but are not limited thereto. The present invention does not place any particular limitations on the formulations above.

Additionally, when preparing eye drops or injections with the pharmaceutical composition according to the present invention, it may be prepared using water for injection. The eye drops or injection may optionally include an isotonic agent, a buffer, an osmotic agent, *etc.* commonly used in the art.

In an embodiment, the present invention provides a health functional food for preventing or improving developmental disorders or neurodegenerative diseases, comprising a crystalline form of the magnesium-serinate compound represented by Formula 1. The functional health food may be usefully used to enhance energy utilization, maintain nerve and muscle function, improve memory, and/or prevent or improve cognitive decline due to aging.

The terms, "developmental disorder" and "degenerative neurological disease" may have the same meaning as described above.

The present invention provides a method for preventing or treating developmental disorders or neurodegenerative diseases, comprising administering to an individual a crystalline form of the magnesium-serinate compound represented by Formula 1 or a pharmaceutical composition comprising the same.

The method for preventing or treating developmental disorders or neurodegenerative diseases of the present invention may comprise administering the crystalline form of the present invention in a therapeutically effective amount.

In the present invention, "administration" means introducing a predetermined material into an individual by an appropriate method.

In the present invention, "individual" means all animals such as rats, mice, livestock, etc., including humans, and may specifically be mammals including humans, but is not limited thereto.

In the present invention, "prevention" refers to all activities that inhibit or delay the onset of a disease by administration of the crystalline form of the present invention.

In the present invention, "treatment" refers to all activities that improve or beneficially change the symptoms of suspected or affected individuals by administration of the crystalline form of the present invention.

In the present invention, "therapeutically effective amount" means an amount that is sufficient to treat a disease at a reasonable benefit/risk ratio applicable to medical treatment and does not cause side effects, which can be determined by those skilled in the art based on patient's sex, age, weight, health condition, type of disease, severity, activity of the drug, sensitivity to the drug, method of administration, time of administration, route of administration, rate of excretion, duration of treatment, drugs used in combination or simultaneously, and other factors well known in the medical field. It is preferable to differently apply a specific therapeutically effective amount for a particular patient according to various factors including the type and extent of the response to be achieved, the specific composition including whether other agents are used as the case may be, the patient's age, weight, general health condition, sex and diet, time of administration, route of administration and secretion rate of the composition, duration of treatment, drugs used together with or concurrently used with the specific composition, and similar factors well known in the medical field.

The pharmaceutical composition and health functional food of the present invention may be prepared through a conventional pharmaceutical composition and a method for preparing health functional food conventional in the art.

The present invention provides a use of a crystalline form of a magnesium-serinate compound for preventing or treating developmental disorders or neurodegenerative diseases.

The present invention provides a use of a crystalline form of a magnesium-serinate compound for the preparation of a medicament for preventing or treating developmental disorders or neurodegenerative diseases.

The matters mentioned in each item of the present invention, that is, the crystalline form, preparation method, pharmaceutical composition, health functional food, treatment method, and use, are equally applied unless they contradict with one another.

### Advantageous Effects of Invention

The crystalline form of the present invention, due to its excellent stability against heat and moisture, does not decompose even when stored for a long period of time, and the crystalline form does not undergo polymorphic transition, and can be stably preserved; therefore, its mass production is easy and economical, and can be usefully used as a pharmaceutical raw material for drugs formulated into oral preparations, injection preparations or eye drops, *etc.*

The crystalline form of the present invention not only has properties advantageous to drug production, but also exhibits bioavailability equal to that of water-soluble L-serine despite its low solubility; therefore, it can be effectively used for the prevention, improvement, or treatment of developmental disorders or neurodegenerative diseases.

### Brief Description of Drawings

Fig. 1 is a drawing showing (a) ¹³C NMR peaks of the magnesium-serinate crystalline form of Example 1 and (b), (c), and (d) theoretical ¹³C NMR peaks according to three structures of the compound in which magnesium and serine are bonded.
Fig. 2 is a diagram showing the six structures of a compound in which six theoretically possible magnesium and serine are bonded.
Fig. 3 is a powder crystal structure of the crystallographic information file (CIF) of the magnesium-serinate crystalline form of Example 1 analyzed using the CIF/PLATON program according to Experimental Example 1.
Fig. 4 is a drawing showing a X-ray powder diffraction pattern of the crystalline form of magnesium-serinate of Example 1.
Fig. 5 is a drawing showing the TG/DSC-MS thermal analysis of the crystalline magnesium-serinate of Example 1.
Fig. 6 is a drawing showing (a) ¹H-NMR and (b) ¹³C-NMR spectra measured by dissolving the magnesium-serinate crystalline form of Example 1 in a D₂O solvent.
Fig. 7 shows images illustrating (a) the properties of the compound of Formula 2 and (b) the crystalline form of the present application according to Experimental Example 7.
Fig. 8 is a graph evaluating the pharmacological effects of Comparative Example 1 and Comparative Example 2 according to Experimental Example 8, and the crystalline form of the present application.

### Mode for the Invention

Hereinafter, examples of the present invention will be described in more detail. However, the following examples are intended to illustrate the present invention, and the scope of the present invention is not limited by these examples, which will be apparent to those skilled in the art.

### <Examples>

### Example 1. Preparation of crystalline form of magnesium-serinate

L-serine (10.53 g) and methanol (50 mL) were added to a round flask and stirred at room temperature for 10 minutes under a nitrogen atmosphere. While stirring the reaction solution at room temperature, magnesium (turnings/chips) (1.21 g) was added thereto, and the reaction solution was vigorously stirred for 90 hours. After filtering through a Büchner funnel, washing with methanol (10 mL), and drying at 110°C for 8 hours, a crystalline form of the magnesium-serinate compound represented by Formula 1 was obtained as a white crystalline solid (11.5 g, 99 % yield). The analysis results of the crystalline form prepared in Example 1 are as follows.

**[Table 1]**

| Test Item | Result |
|---|---|
| Heavy metal | 20 ppm or less |
| Content | 98.6 % |

A heavy metal test was performed according to Method 1 of the Heavy Metal Test Methods, a general test method of the 12th revision of the Korean Pharmacopoeia. In the content test, about 200 mg of the sample prepared in Example 1 was precisely quantified and dissolved in 50 mL of water, and about 2 mL of ammonia/ammonium chloride buffer (pH 10.7) and 3 to 4 drops of Eriochrome Black T indicator were added thereto and then titrated with 0.05 M-EDTA·2Na. The end point of the titration was determined as the time when the red color changes to blue and persists for at least 30 seconds. In the same way, a blank test was performed and corrected, and then the content was calculated.

### Example 2. Preparation of crystalline form of magnesium-serinate

85 g of a magnesium methoxide solution (a Mg content of 8 wt% to 9 wt%) was added to a round flask, L-serine (10 g) was added thereto, and the mixture was refluxed and stirred for 4 hours. Then, the reaction solution was cooled to room temperature, filtered through a Büchner funnel, washed with about 30 g of methanol, and dried under vacuum at 60 °C for 12 hours or more to obtain a crystalline form of the magnesium-serinate compound represented by Formula 1 as a white crystalline solid (10.92 g, 99 % yield). The prepared crystalline form was identified through PXRD, DSC, and NMR to confirm substantially the same results as in Example 1. The analysis results of the prepared Example 2 are as follows.

**[Table 2]**

| Test Item | Result |
|---|---|
| pH | 10.3 |
| Heavy metal | 20 ppm or less |
| Moisture | 0.1% |
| Content | 100.0% |

Moisture and pH evaluations were performed in the same manner as in Experimental Example 7, and the heavy metal test and the content test were performed in the same manner as in Example 1.

### Experimental Example 3. Preparation of crystalline form of magnesium-serinate

After adding methanol (150 mL) into a round flask, L-serine (10 g) and magnesium ethoxide (5.44g) were added thereto, and the mixture was refluxed and stirred for about 5 hours. Then, the reaction solution was cooled to room temperature, filtered through a Büchner funnel, washed with about 30 g of methanol, and dried under vacuum at 60 °C for 12 hours or more to obtain a crystalline form of the magnesium-serinate compound represented by Formula 1 as a white crystalline solid (11.03 g, 100 % yield). The prepared crystalline form was identified through PXRD, DSC, and NMR to confirm substantially the same results as in Example 1.

### <Experimental Examples>

Hereinafter, AST-011 (Comparative Example 2) was prepared in the same manner as described in Example 1.1 of Korean Patent No. 10-1952443.

### Experimental Example 1. Analysis of crystalline form

The structure determination from powder diffraction (SDPD) method was used to identify the crystalline structure of the magnesium-serinate compound represented by Formula 1 of the present invention. The SDPD method requires high-quality powder XRD data. The initial structure candidates were identified through several manipulation steps, such as baseline correction, peak searching, and indexing of the data, and the structures were refined through Rietveld refinement, and finally, the crystal structure was confirmed by performing validation on the refined structure. The SDPD method includes three methods: a direct method, a real space method, and a charge flipping method, and in the present invention, structural analysis was performed using the real space method, which utilizes actually measured PXRD data. The real space method involves confirming whether the resulting structure aligns with physicochemical theoretical values by matching the measured values and theoretically derived PXRD simulation values using the least squares method, while comparing the actually measured PXRD data with theoretical PXRD data regarding the structure of the basal value.

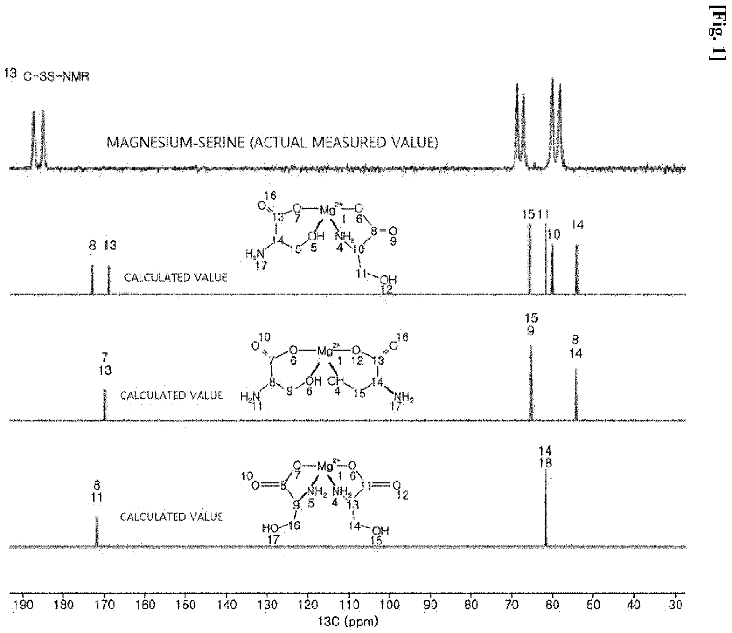

In order to obtain the structure of the basal state candidate material, information on how the Mg atom and two L-serine molecules coordinate according to the crystalline structure was analyzed through solid-state NMR. As shown in Fig. 1, the theoretical ¹³C NMR peaks of the three types of compounds in which magnesium and serine are bonded according to the bonding structure, are different. This model appears similar to solid-state NMR because it is an expected spectrum when nitrogen or oxygen is coordinated. In general, simulations for liquid samples are not distinguished because they are not used unless they are strong coordination models due to their high degree of freedom.

When SS ¹³C-NMR (solid-state ¹³C-NMR) (Fig. 1a) of the magnesium-serinate crystalline form of the present invention was measured, it was confirmed that a result similar to that of the pentagonal-hexagonal coordination compound (Fig. 1b) was obtained. Considering that the same amount of the hexagonal-hexagonal (Fig. 1c) and pentagonal-pentagonal (Fig. 1d) compounds are present at the same time, three peaks around 60 ppm would appear.

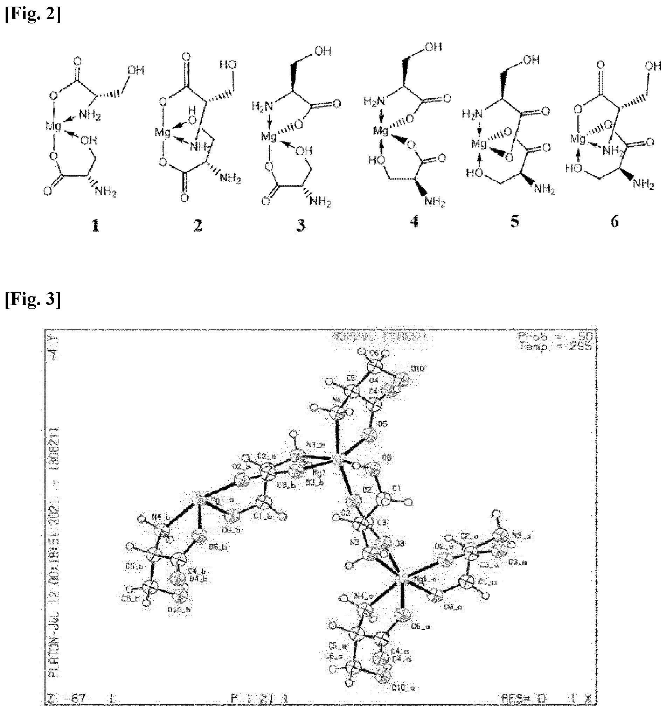

From these experimental results, pentagonal-hexagonal compounds may be selected as basal-state candidate materials, and the number of such compounds may be six structures as shown in Fig. 2.

In order to confirm the magnesium-serinate of the present invention among the six candidate crystal structures, the structure was sequentially established by the SDPD method and the analysis was performed accordingly.

The PXRD data were processed through background setting and peak search using the EXPO2014 program, followed by indexing and space group determination using the built-in N-TREOR90 algorithm. The integrated intensities were then obtained using the Le Bail method, and an approximate crystalline structure was derived using a reciprocal space method (Simulated Annealing), and finally, the definitive crystal structure, represented as Formula 1, was obtained through Rietveld refinement.

Fig.3 shows the confirmation of the crystalline structure of the magnesium-serinate crystal form represented by Formula 1 of the present invention, which was achieved using the PLATON program by uploading to the CheckCIF website (http://checkcif.iucr.org) provided by the IUCr, using the Crystallographic Information File (CIF), which is a standardized data format of crystallography information developed under the leadership of the Working Party on Crystallographic Information (WPCI) of the International Union of Crystallography (IUCr).

### Experimental Example 2. Analysis of X-ray powder diffraction (PXRD)

The X-ray powder diffraction (PXRD) analysis of the magnesium-serinate crystalline form prepared in Example 1 of the present invention was performed according to the analysis conditions and measuring instrument specifications shown in Table 3 below. The main X-ray diffraction pattern diffraction angles (2θ±0.2°) and relative intensities are shown in Table 4 and Fig. 4 below. In Fig. 4, the x-axis is 2θ (Bragg angle, unit: °).

**[Table 3]**

| | | | |
|---|---|---|---|
| Instrument | Rigaku SmartLab Bragg-Brentano diffractometer | | |
| | X-Ray generator | Max. power 9 kW or higher | |
| | Incident K-alpha-1 optics monochromator | Optics | Symmetric Johansson Ge(111) curved crystal |
| | | X-ray wavelength | Cu K-alpha-1 |
| | High precision goniometer | Geometry | Vertical θ/θ geometry with horizontal sample mounting |
| | | Minimum step size | 0.0001° or smaller |
| | | Reproducibility | 0.0001° or better |
| | | 2theta range | -3 to 160° |
| | | Goniometer radius | 300 mm or longer |
| | Detectors | 2D detector (Hypix-3000) & High speed | |
| | | 1D detector (D/teX Ultra 250) | |
| | Attachment | Atmosphere separator | |
| | Power | 45 kV 200 mA | |
| Condition | 2θ-θ range scan speed | 5°-65° | |
| | | 0.4°/min | |

**[Table 4]**

| **Angle 2θ (°)** | **d value (Å)** | **Intensity (count)** | **Intensity (%)** |
|---|---|---|---|
| 9.122 | 9.68655 | 685 | 9.3 |
| 13.534 | 6.53735 | 7352 | 100 |
| 15.996 | 5.53612 | 5184 | 70.5 |
| 18.361 | 4.82822 | 1468 | 20 |
| 18.892 | 4.69351 | 757 | 10.3 |
| 19.747 | 4.4922 | 1936 | 26.3 |
| 19.995 | 4.43716 | 1121 | 15.2 |
| 20.951 | 4.23681 | 1728 | 23.5 |
| 22.048 | 4.02828 | 6019 | 81.9 |
| 24.387 | 3.64706 | 1168 | 15.9 |
| 25.732 | 3.4593 | 953 | 13 |
| 26.39 | 3.37458 | 860 | 11.7 |
| 27.302 | 3.26387 | 641 | 8.7 |
| 28.372 | 3.14322 | 1317 | 17.9 |
| 29.549 | 3.02063 | 923 | 12.6 |
| 30.067 | 2.96975 | 723 | 9.8 |
| 31.306 | 2.85499 | 569 | 7.7 |
| 32.387 | 2.76213 | 916 | 12.5 |
| 33.51 | 2.67203 | 839 | 11.4 |
| 33.985 | 2.63578 | 792 | 10.8 |
| 34.417 | 2.60367 | 995 | 13.5 |
| 36.101 | 2.48602 | 563 | 7.7 |
| 36.768 | 2.4424 | 474 | 6.4 |
| 38.372 | 2.34395 | 502 | 6.8 |
| 40.362 | 2.23286 | 724 | 9.8 |
| 40.925 | 2.20343 | 605 | 8.2 |
| 42.734 | 2.11423 | 436 | 5.9 |
| 43.295 | 2.08814 | 454 | 6.2 |
| 44.285 | 2.04373 | 473 | 6.4 |
| 44.722 | 2.02477 | 462 | 6.3 |
| 46.494 | 1.95161 | 360 | 4.9 |
| 47.092 | 1.92821 | 342 | 4.7 |
| 48.301 | 1.88277 | 311 | 4.2 |
| 50.61 | 1.80212 | 285 | 3.9 |
| 52.906 | 1.7292 | 270 | 3.7 |
| 53.839 | 1.70142 | 297 | 4 |
| 55.842 | 1.64505 | 250 | 3.4 |
| 58.753 | 1.5703 | 205 | 2.8 |
| 62.417 | 1.48663 | 209 | 2.8 |

Referring to Table 4 above and Fig. 4, it can be confirmed that the crystalline form of the present invention is a novel crystalline compound and is a different crystalline compound from the amorphous AST-011 (Comparative Example 2).

### Experimental Example 3. Analysis of differential scanning calorimetry (DSC)

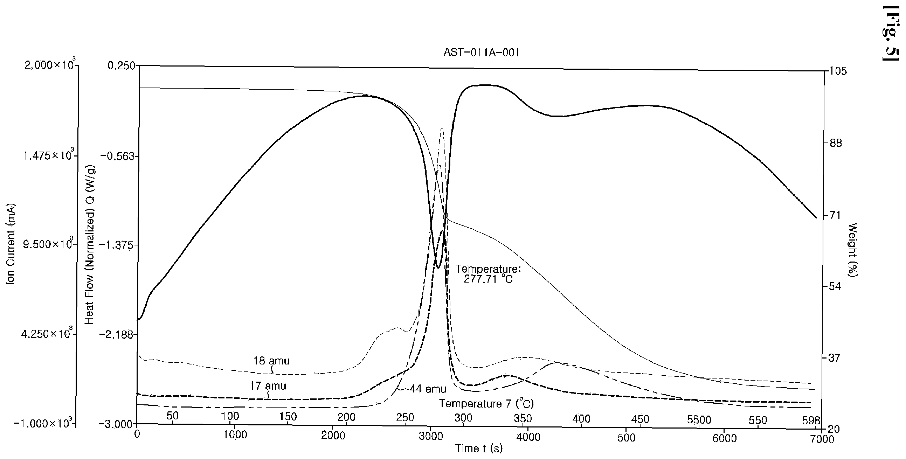

For the analysis of the crystalline form of the present application (Example 1) by differential scanning calorimetry, the measurement was performed while raising the temperature from 0 °C to 500 °C at a heating rate of 10 °C/min using a DSC-N650 (SCINCO, Korea) as a DSC analyzer. The maximum endothermic peak temperature of the crystalline form obtained through differential scanning calorimetry (DSC) analysis is shown in Fig. 5.

Referring to Fig. 5, it can be seen that the crystalline form of the present application exhibits the maximum endothermic peak at 283±3 °C. That is, it can be confirmed that the crystalline form of the present invention exhibits new thermodynamic properties different from AST-011 (Comparative Example 2), which does not show a main endothermic peak, and is a new thermodynamically stable crystal.

### Experimental Example 4. Analysis of nuclear magnetic resonance (NMR)

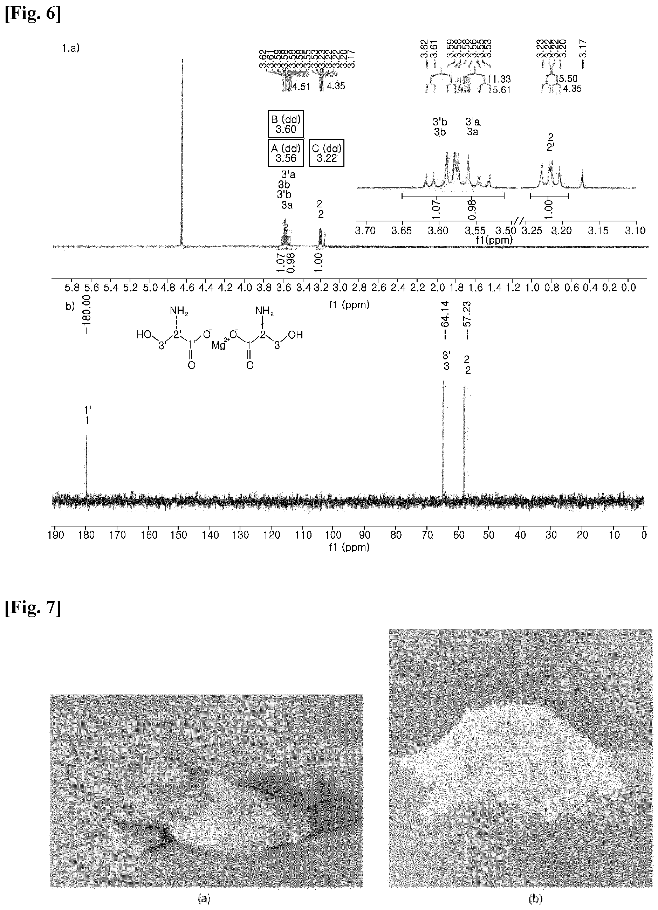

¹H NMR and ¹³C NMR analyses of the crystalline form of the present application prepared in Example 1 were performed. 20 mg of the crystalline sample of the present application was dissolved in 0.7 mL of D₂O, and the resultant was subjected to nuclear magnetic resonance analysis using JEOL/400YH (JEOL, USA) at 25 °C, and the results are shown in Fig. 6.

### Experimental Example 5. Stability test under harsh condition

In order to confirm the stability of the crystalline form of the present invention depending on conditions, a stability test was conducted under harsh conditions.

The magnesium-serinate crystalline form prepared in Example 2 was stored in a solid state at 70 °C and in an aqueous solution having a concentration of 0.5 mg/mL at 25 °C (60.0 % relative humidity) for 21 days, respectively, and then maleic acid was set as a standard substance, and the stability (measurement of content and related materials) was confirmed through qNMR analysis.

For the qNMR analysis method, analysis conditions were established by measuring the spin-lattice relaxation time T1 of the nuclide ¹H of magnesium-serinate on the sample obtained through the preliminary stress test. The T1 value was set at 4.708 seconds, and the measurement waiting time (d1: delay time) and measurement time (at: acquisition time) were set based on this value. The measurement waiting time (d1) was set to 30 seconds, and the measurement time (at) was set to being equal to or greater than the T1 value.

For the ¹H-NMR measurement for qNMR analysis, JEOL's JNM-ECX400I (400 MHz) NMR equipment was used, and spectral width (sw) was set at 6,000 Hz to sufficiently reflect the frequency range from 0 ppm to 10 ppm.

The analysis results are shown in Table 5 below.

**[Table 5]**

| Condition | Purity of magnesium-serinate (%) | | Concentration of Total Related Materials (%) | |
|---|---|---|---|---|
| | initial state | 9 days | initial state | 21 days |
| 70°C, Solid state | 100.12 | 100.15 | 0.00 | 0.00 |
| 25°C, Aqueous solution state | 100.12 | 100.07 | 0.00 | 0.00 |

As can be seen from the results of Table 5 above, it was confirmed that the magnesium-serinate crystalline form of the present invention was very stable in both solid and aqueous solutions even under harsh conditions, and no decomposition products and related materials were generated. Therefore, it was confirmed that the crystalline form of the present invention can be used very usefully as a pharmaceutical raw material.

### Experimental Example 6. Evaluation of occurrence of polymorphic transition

In order to confirm whether the polymorphic transition of the magnesium-serinate crystalline form of the present invention occurs over time, PXRD and DSC analyses were performed in the initial state and after 4 weeks to confirm whether or not the polymorphic transition occurred.

Specifically, the crystalline form prepared in Example 2 was added into a polyethylene bag, placed in a stability chamber under conditions of a temperature of 40 °C and a relative humidity of 75 %, and stored for 4 weeks. The results of PXRD and DSC measured at the beginning and after 4 weeks, respectively, are shown in Table 6 below. PXRD and DSC analyses were performed in substantially the same manner as in Experimental Examples 2 and 3.

**[Table 6]**

| Category | Crystalline form of magnesium-serinate of the present application | |
|---|---|---|
| | initial | after 4 weeks |
| DSC Endothermic Temperature | 283.0°C (dec.) | 282.5°C (dec.) |
| Result of PXRD Analysis | same crystalline form | |
| Result of Comparative Analysis | no occurrence of polymorphic transition | |

As can be seen from the results of the PXRD and DSC analyses in Table 6 above, the magnesium-serinate crystalline form of the present invention did not undergo polymorphic transition even after 4 weeks of storage. That is, it was confirmed that the magnesium-serinate crystalline form of the present invention is a very stable crystalline form in which polymorphic transition does not occur due to aging and can be used very usefully as a pharmaceutical raw material.

### Experimental Example 7. Comparison of crystalline form of the present application and amorphous AST-011 (Comparative Example 2)

The crystalline form of the present application prepared in Example 1 and the amorphous form of AST-011 (Comparative Example 2) were comparatively evaluated for the following test items by the following methods, and the results are shown in Table 7 below.
- Appearance evaluation: Visual observation
- Moisture measurement: According to the moisture measurement method (Karl Fischer method) of the general test method of the 12th edition of the Korean Pharmacopoeia, measured by adding 0.5 g of the sample into a moisture meter (Mettler Toledo, V10-S, Switzerland).
- Infrared spectrum measurement method (IR): Measurement was made with FT-IR equipment (PerkinElmer, Spectrum 2, USA) according to the ATR measurement method of the general test method of the 12th revision of the Korean Pharmacopoeia.
- pH measurement: 0.5 g of the sample was added into a 10 mL volumetric flask, and water was added to a final volume of 10 mL, and measurement was made with a pH meter (Mettler Toledo, FP-20 Switzerland).
- XRD and DSC analyses: The analyses were performed in the same way as in Experimental Examples 2 and 3.

**[Table 7]**

| Test Item | AST-011 (Comparative Example 2) | Crystalline Form of Present Application |
|---|---|---|
| Moisture Content | 8.6% | 0.2% |
| IR | same functional group as serine | same functional group as serine |
| pH | 9.5 | 10.3 |
| DSC | no endothermic peak | endothermic peak at 283.7 °C |
| XRD | no crystallinity | serine, exhibits different crystallinity from magnesium hydroxide |
| Appearance | Fig. 7(a) a material with white viscosity (when stored, appearance changes within 7 days) | Fig. 7(b) light gray or white powder (stability: no change after 3 months) |

As can be seen from Table 7 above, it was confirmed that AST-011 (Comparative Example 2) is an amorphous compound with a high moisture content and no main endothermic peak, and it is an unstable compound that shows a change in appearance when stored for 7 days. In contrast, it was confirmed that the crystalline form of the present application is a stable crystalline compound having a low moisture content and an endothermic peak at 283±3°C, and is a stable compound with no change in properties even after 3 months of storage under the same conditions as AST-011.

That is, it was confirmed that the crystalline form of the present invention can be used very usefully in a mass production process, *etc.* as a pharmaceutical raw material.

### Experimental Example 8. Test of pharmacological effects

A 1-day-old mouse brain was removed, the olfactory bulb was removed, and the meninges were peeled off. After collecting the resultant in a 50 mL tube including HBSS (Hank's balanced salt solution) and centrifuging at 250 × g for 1 minute, the supernatant was removed. 2 mL of trypsin/EDTA (0.5 %), 8 mL of HBSS buffer, and 0.2 mL of DNase were mixed and reacted in a 37 °C constant temperature water bath for 30 minutes. After adding 18 mL of the culture medium and 2 mL of trypsin/EDTA thereto and pipetting 10 times, the resultant was filtered using a 40 µm cell strainer. Then, the supernatant was removed after centrifugation at 800 × g for 2 minutes. After adding 5 mL of the culture solution thereto, the number of cells was confirmed using a hemocytometer and the cells were cultured to obtain primary mouse astrocytes.

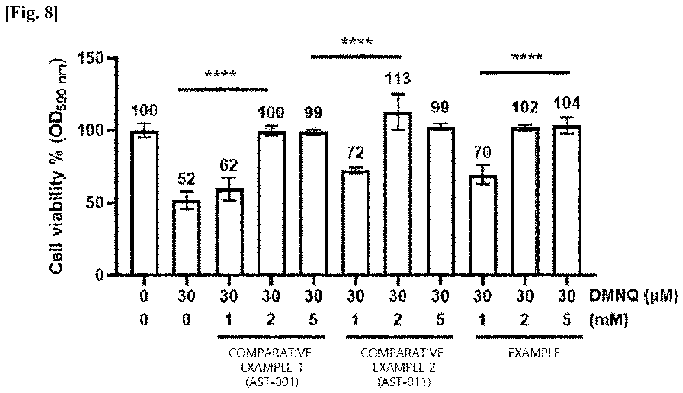

The cytoprotective effect of L-serine (AST-001, Comparative Example 1), AST-011 (Comparative Example 2), and the crystalline form of the present application (Example 1) on oxidative stress induced to these cells by DMNQ (1,4-naphthoquinone) was confirmed. After inoculating cells (primary astrocytes, 1 × 10⁵ cells) per well in a 96-well plate and culturing at 5 % CO₂ and 37 °C for 24 hours to obtain a cell monolayer, the composition was diluted with a cell inoculation medium and the diluted composition each at a concentration of 0 mM, 1 mM, 2 mM, and 5 mM was inoculated at 100 µL/well, and then cultured in a 5 % CO₂ and 37 °C incubator for 6 hours. After removing the culture medium, 0 mM, 1 mM, 2 mM, and 5 mM of DMNQ (30 µM) causing cytotoxicity, each of the compounds of Comparative Example 1 and Comparative Example 2, and the crystalline form of Example 1 of the present application were mixed and inoculated at 100 µL/well, respectively, and then cultured in a 5 % CO₂ and 37 °C incubator for 16 hours. Then, after removing the culture medium, 0.5 mg/mL of MTT solution was added to each well in an amount of 100 µL and reacted in a 5 % CO₂ and 37 °C incubator for 2 hours. After completely removing the MTT solution and drying the culture plate, the formazan crystals formed in the cells were dissolved with DMSO, and the absorbance was measured at 590 nm with an ELISA plate reader to evaluate cell viability and drug efficacy. The results are shown in Fig. 8.

As can be seen through Fig. 8, about 50 % of the cells were killed by oxidative stress induced by DMNQ. In contrast, in the group pre-treated with the compound of Comparative Example 1 (AST-001) or Comparative Example 2 (AST-011) or the crystalline form of the present application followed by post-treatment with DMNQ, the cell viability was excellent due to the cell protective effect of the drug, thus confirming their pharmacological effects. It was confirmed that the cytotoxic protective effect of the crystalline form of magnesium-serinate of the present invention on DMNQ was similar to that of L-serine and AST-011, thus confirming that the crystalline form of the present invention has a pharmacological effect that is within a substantially equivalent range to those of L-serine and AST-011.

According to the experimental results above, it was confirmed that the magnesium-serinate crystalline form of the present invention has excellent stability, does not undergo polymorphic transition over time, and has excellent pharmacological effects, and thus is optimized as a pharmaceutical raw material.

While the specific parts of the present invention have been described in detail above, it is obvious to those skilled in the art that such specific descriptions are merely preferred embodiments and that the scope of the present invention is not limited thereto. Accordingly, the actual scope of the present invention will be defined by the accompanying claims and equivalents thereof.

## Claims

1. A crystalline form of a magnesium-serinate compound represented by Formula 1 below:

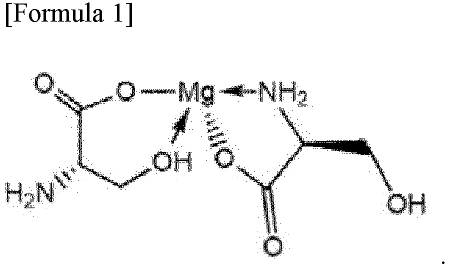

2. The crystalline form of a magnesium-serinate compound of claim 1, wherein the X-ray powder diffraction pattern comprises:
diffraction peaks at diffraction angles (2θ±0.2°) of 13.53°, 16.00°, and 22.05°; and
three or more diffraction peaks at diffraction angles (2θ±0.2°) selected from the group consisting of 18.36°, 19.75°, 20.00°, 20.95°, 24.39°, and 28.37°.

3. The crystalline form of a magnesium-serinate compound of claim 2, wherein the X-ray powder diffraction pattern further comprises one or more diffraction peaks at diffraction angles (2θ±0.2°) selected from the group consisting of 18.89°, 25.73°, 26.39°, 29.55°, 30.07°, 32.39°, 33.51°, 33.99°, 34.42°, and 40.36°.

4. The crystalline form of a magnesium-serinate compound of claim 1 having an endothermic peak at 283±3°C when analyzed by differential scanning calorimetry (DSC).

5. A crystalline form of a magnesium-serinate compound in which two serine molecules are bound to one atom of magnesium,
wherein the X-ray powder diffraction pattern comprises diffraction peaks at diffraction angles (2θ±0.2°) of 13.53°, 16.00°, and 22.05°.

6. The crystalline form of a magnesium-serinate compound of claim 5 having an endothermic peak at 283±3°C when analyzed by differential scanning calorimetry (DSC).

7. A method for preparing a crystalline form of the magnesium-serinate compound according to any one of claims 1 to 6, comprising:
a) mixing L-serine, magnesium (Mg) or magnesium oxide, and a solvent and stirring the mixture; and
b) filtering the crystals produced after the stirring.

8. The method of claim 7, wherein the magnesium (Mg) is in powder or granular form.

9. The method of claim 7, wherein the magnesium oxide is one or more selected from the group consisting of magnesium methoxide, magnesium ethoxide, magnesium chloride, magnesium bromide, magnesium carbonate, and magnesium citrate.

10. The method of claim 7, wherein the solvent is a polar solvent.

11. The method of claim 7, wherein the solvent is one or more selected from the group consisting of methanol, ethanol, isopropanol, and t-butanol.

12. A pharmaceutical composition for preventing or treating developmental disorders or neurodegenerative diseases comprising the crystalline form of the magnesium-serinate compound according to any one of claims 1 to 6 as an active ingredient.

13. The pharmaceutical composition of claim 12, wherein the developmental disorders are one or more selected from the group consisting of autism spectrum disorder (ASD), attention deficit hyperactivity disorder (ADHD), intellectual disability, language disorder, motor disorder, tic disorder, and Tourette syndrome.

14. The pharmaceutical composition of claim 12, wherein the neurodegenerative diseases are one or more selected from the group consisting of Alzheimer's disease, vascular dementia, mild cognitive impairment, Parkinson's disease, Huntington's disease, amyotrophic lateral sclerosis, multiple sclerosis, and cerebellar degenerative diseases.

15. The pharmaceutical composition of claim 12, wherein the pharmaceutical composition is formulated into one or more formulations selected from the group consisting of powders, granules, tablets, capsules, suspensions, emulsions, syrups, aerosols, suppositories, eye drops, and injections.

16. A health functional food for preventing or improving developmental disorders or neurodegenerative diseases comprising the crystalline form according to any one of claims 1 to 6.

17. A method for preventing or treating developmental disorders or neurodegenerative diseases, comprising administering, to an individual, the crystalline form of a magnesium-serinate compound according to any one of claims 1 to 6.

18. Use of the crystalline form of a magnesium-serinate compound according to any one of claims 1 to 6 for preventing or treating developmental disorders or neurodegenerative diseases.

19. Use of the crystalline form of a magnesium-serinate compound according to any one of claims 1 to 6 in preparation of a medicament for preventing or treating developmental disorders or neurodegenerative diseases.
